# EUROPEAN PATENT APPLICATION

(11) **EP 3 851 104 A1**
(43) Date of publication of application: **21.07.2021**
(21) Application number: 19860849.9
(22) Date of filing: 12.09.2019
(51) Int. Cl.: A61K 31/415, A61K 31/122, A61K 31/192, A61P 19/02

(54) **SYNERGIC PHARMACEUTICAL COMBINATION OF A SELECTIVE INHIBITOR OF CYCLOOXYGENASE-2 AND AN ANTHRAQUINONE DERIVATIVE**

(30) Priority: 13.09.2018 MX 2018011142
(71) Applicant: Amézcua Amézcua, Federico, Guadalajara, Jalisco, 44898 (MX); Amézcua Amézcua, Carlos, Guadalajara, Jalisco, 44898 (MX)
(72) Inventor: GARCÍA ARMENTA, Patricia del Carmen, Jalisco, 45019 (MX); CHÁVEZ GARCÍA, José Alonso, Jalisco (MX)
(74) Representative: Ungria López, Javier
(86) International application number: PCT/MX2019/000097
(87) International publication number: WO 2020/055226

(57) **Abstract**

The present invention relates to a pharmaceutical composition comprising a synergic combination of a selective inhibitor of cyclooxygenase-2 (COX-2), such as the active substance celecoxib, and an agent derived from anthraquinone, such as the active substance diacerein, which are formulated with pharmaceutically acceptable additives and/or excipients and/or vehicles in a single dosing unit to be administered orally, parenterally, topically, transdermally or by devices for oral or nasal inhalation. The composition is suitable for the treatment of pain and inflammation in osteoarthritis, rheumatoid arthritis and/or degenerative joint disease caused by different aetiologies.

## Description

### FIELD OF INVENTION

This invention is connected with the technical field of the pharmaceutical industry, as its purpose is to provide a pharmaceutical composition comprising of the synergistic pharmaceutical combination of a selective inhibitor of cyclooxygenase-2, made up by the active ingredient celecoxib or its pharmaceutically acceptable salts and an anthraquinone derivative made up by the active ingredient diacerein or one of its pharmaceutical acceptable salts, which are administered with pharmaceutically acceptable excipients or adjuvants, formulated in a single dosing unit to be administered orally, for long-term pharmacological use in the treatment of pain and inflammation in osteoarthritis, rheumatoid arthritis and/or degenerative joint disease. Said combination has enhanced properties and an additional antiarthritic effect.

The combination of the aforementioned active ingredients produces a stronger therapeutic effect when they are administered together in a single dosage unit, unlike when they are administered separately, providing the benefits of a smaller dose being required, higher therapeutic effect and fewer adverse effects.

### BACKGROUND

Rheumatoid arthritis (RA) is a chronic inflammatory disease that affects joints symmetrically (the joints on both sides of the body), but can also damage internal organs, which is why should be treated as a systemic disease, in other words a disease that affects the entire body. The inflammation of joints, or better said arthritis, is produced because some immune system cells (lymphocytes) attack the synovial membrane. If the inflammation continues it can, over time, lead to joint destruction and progressive disability.

Rheumatoid arthritis is a chronic autoimmune disease that is spread throughout the world and, apart from some exceptions, characterized by its strong impact on the functionality of people, owing to the inflammatory activity it generates in their joints. However, it has not been possible to demonstrate that the factors that determine its occurrence are the same in every society, moreover, the clinical expression of the disease differs between populations. Its prevalence varies and there is an estimated range of between 0.2% and 5% in the world.

Rheumatoid arthritis produces pain, swelling, reddening and increased temperature in the affected joints. Sometimes only the rheumatologist can detect this swelling by palpating joints or through the use of tests such as echography or magnetic resonance. Not all joints are affected with the same frequency. Wrists, knuckles, finger and toe joints, elbows, shoulders, hips, knees and ankles are the joints that are commonly inflamed. Rheumatoid arthritis can also cause neck pain. Moreover, it can be hard to start moving in the morning (morning stiffness) for more than half an hour. If the inflammation persists it can end up damaging the surrounding bone, ligaments and tendons. The consequence shall be the progressive deformity of joints and the reduction of joint mobility, which can lead the patient to a certain degree of disability for performing some tasks of daily life. Furthermore, it is worth remembering that rheumatoid arthritis can produce symptoms of its damage unconnected with the joints, such as, for example: inexplicable fever, fatigue, pins and needles in hands or feet, ongoing hoarseness without the patient having catarrh, shortness of breath, continuous cough, chest pain or pain in the sides.

Apart from rheumatoid arthritis, osteoarthritis (OA) is considered to be the most common of the chronic rheumatic diseases. It appears in the form of pain, deformity and functional disability mainly of weight-bearing and highly-mobile joints. With the change in the epidemiological panorama to chronic-degenerative diseases and the ageing of the world's population, osteoarthritis is a public health problem must be effectively attended to in a timely manner.

OA, also known as arthrosis or osteoarthrosis, is a chronic degenerative disease that is characterized by the gradual and progressive destruction of the cartilage that coats the articular surface of knees, hips, shoulders, hands, ankles and the spinal column. Furthermore, there is inflammation of the synovial membrane as well as damage to menisci, tendons, muscles and nerves associated with the affected joint.

Arthrosis is a highly prevalent joint chronic disease that particularly appears in the elderly. Pain is one of the main symptoms and the major determining factor for the loss of functionality, and the symptoms persist in a lot of patients despite the usual treatments.

OA is treated as a public health problem owing to its high rate of incidence and prevalence. At least 15% of 60-year-olds have it worldwide. It has even been found that, of all the rheumatic diseases, OA is 10 to 12 times more common than rheumatoid arthritis.

OA in the knee is the most clinically significant and increases with age. 33% and 53% of men and women, respectively, over 80 years of age have radiological evidence of OA, however, the clinical symptoms are only reported in 16% of women and 5% of men who are over 80 years of age.

The 1998 national health survey (ENSA II) in Mexico found it to be the second cause of morbidity with 14% in people over 60 years of age.

The incidence of OA of the hip is 88/100,000 people/year, OA of the knee is 240/100,000 people/year. The incidence of OA of the knee is 1% per year in women between 70 and 89 years of age.

The studies carried out to determine the primary care costs of musculoskeletal diseases report that OA holds the first four places in demand for care and represents the same level in terms of costs.

It is a complaint that primarily compromises the articular cartilage with progressive tendencies and different etiologies. During its evolution, it can end up affecting the complete joint including subchondral, synovial, capsule bone, periarticular ligaments and muscles.

There is a consensus that the prevalence of osteoarthrosis increases with age, although degenerative joint changes can be demonstrated from the second decade of life and some load abnormalities in the majority of 40-year-olds. Then their frequency increases from that age on and is to be found in everyone who is 75 years of age and older. It affects both sexes equally and racial differences only relate to its location (coxofemoral, in particular).

The related etiological factors are diverse. Neither climate nor geography have an influence. Being overweight has an influence on weight-bearing joints and jobs and activities such as sports and habits of posture also have an influence. One thing though that is generally agreed is that age is not the sole determining factor.

There is a progressive loss of articular cartilage and its deterioration gives rise to reactive changes in the joint margins and the subchondral bone.

Osteoarthrosis develops under two conditions: when the biostructural properties of the cartilage and the subchondral bone are normal but excessive joint loads induce tissue changes; or when the load is reasonable but the cartilaginous and bone structure are deficient.

The cartilage is eroded and destroyed, does not regenerate and can disappear, if not in its entirety, significantly and extensively. The subchondral bone responds by giving rise to the production of "new bone " and the resulting marginal osteophytes can be seen from the outside as nodules that can be secondarily inflamed or as growth bone capable of irritating neighboring structures (radiculitis, for example, in the case of osteophytes that close the intervertebral foramina).

Biochemical cartilaginous changes affect both proteoglycans and type II collagen and, in the advanced stages, the chondrocytes are incapable of compensating the loss of proteoglycans and the loss occurs of cartilaginous matrix. The enzymatic family that is identified as harmful is the de the metalloproteinases, the serine and thiol proteases. Moreover, the cytokines participate as mediators in tissue damage (interleukins and tumor necrosis factors).

The dominant symptom in osteoarthrosis is joint pain, which is relieved by rest, but increases when activity is renewed. Osteoarthritic knee pain that is exaggerated when starting to walk after rest and is relieved after a little walking is typical. Later, the pain can be spontaneous and even during rest at night. Inflammation only exists as an additional factor, a complication, giving rise to inflammatory outbreaks in addition to the painful joint. The causes of these outbreaks may be unidentifiable, evidently post-traumatic or because of the deposit of calcium crystals (pyrophosphate) and sometimes the synovial inflammatory process can lead to hydrarthrosis.

Associations of analgesic agents containing two or more compounds are widely used in therapeutics, many of these associations are formulated in an effort to produce better painkilling effects.

Despite the fact that the effect of symptomatic slow acting drugs (SYSADOA "Symptomatic Slow Acting Drugs for Osteoarthritis") has a slow start, as additional advantages they have an overall effectiveness similar to that of NSAIDs and an effect lasts longer, even for some months after the treatment is withdrawn (carry over effect) and the anti-arthrosic properties owing to their ability to inhibit pro-inflammatory and pro-catabolic cytokines such as interleukin-1, has a slow onset of action that is not significant until after 4 weeks but continues for 2 months once the treatment is suspended.

At the present time, there are rheumatoid arthritis treatments that include pharmacological treatment. The combination of celecoxib with diacerein is an association that has potential therapeutic utility in patients that suffer pain.

Many nonsteroidal anti-inflammatory painkillers (NSAIDs) are employed in the pharmacological treatment of pain. Nowadays there is also a preference for the use of selective cyclooxygenase-2 inhibitor drugs, such as celecoxib, that have shown themselves to be useful in the treatment of a variety of pain conditions and cause fewer adverse gastrointestinal (GI) effects. unlike NSAIDs, diacerein can stimulate or not affect the synthesis of PGs, and acts by inhibiting interleukin-1, so it has a potential utility as a possible non-ulcerogenic alternative to NSAIDs for the treatment of patients with abnormalities such as osteoarthritis.

Diacerein is a anthraquinone derivative compound with an unusual mechanism of action. Both diacerein and particularly its active diacetylated derivative "rhein", are IL-1 inhibitors, that have shown antinociceptive, antipyretic and anti-inflammatory activity, affecting the pain threshold in rats with edema in a limb and hyperpyrexia in rabbits. Diacerein and its metabolite specifically inhibit the production of interleukin 1 beta in in vitro human monocytes. It is known that even when it inhibits the IL-1 that mediates the production of collagenase in articular cartilage, it does not alter the kidney or platelet cyclooxygenase (COX) activity, however, the exact mechanism of the therapeutic action of diacerein is not clear yet.

The PIFIR experimental model of the joint authors, López-Muñoz and collaborators, is a preclinical model where it is possible to establish gout in rats and compare the antinociceptive effectiveness of a variety of individual drugs or drugs in combination in the animals with this alteration. This experimental model has some significant advantages over other preclinical methodologies: a) establishes an alteration in rats that clinically affects humans: gout; b) the temporary course of the analgesic or antinociceptive effect can be determined for up to 4 continuous hours in the same experimental subject, without producing conditioning or learning in the rat, which is a significant advantage in comparison with other experimental models; c) less test compound is consumed than in other experimental models that use larger animal species; d) the assessment of the antinociceptive effects is done by means of a computer and does not depend on the sometimes subjective observations of the assessor. As already mentioned, the PIFIR model has been very useful for studying the antinociceptive effects of analgesic compounds by themselves as well as of combinations of analgesic compounds, for studying the mechanisms of action of analgesic compounds.

Celecoxib is the compound 4-[5-(4-methylphenyl)-3-(trifluoromethyl)-1*H-*pyrazol-1-yl]benze nesulfonamide, represented by the formula (I)

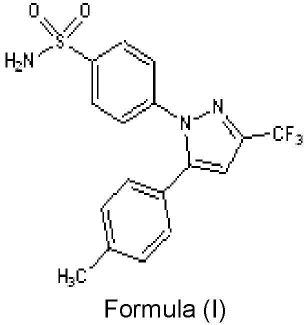

Described for the first time in US 5,466,823 for the treatment of the inflammation and disorders associated with inflammation, such as arthritis.

Celecoxib is only administered orally; food does not interfere with its absorption and maximum plasma concentration is reached around 3 hours. The approved daily doses are: from 100 to 400 mg; it is a selective cyclooxygenase-2 inhibitor (COX-2). It 97% bonds to the plasma proteins and suffers extensive hepatic metabolism, by the CYP2C9 isoenzyme of the P-450 cytochrome. It is indicated for the symptomatic relief of arthrosis and rheumatoid arthritis. Its effectiveness has been assessed through a variety of clinical trials, including comparisons with other NSAIDs: diclofenac, naproxen and ibuprofen, showing more effectiveness than the placebo and similar to that of these agents.

Celecoxib presents high selectivity for COX-2 receptors, does not present COX-1 inhibitor activity at therapeutic concentrations unlike non-selective NSAIDs, it does not significantly affect platelet aggregation or hemorrhage time.

There is a proposed advantage of selective COX-2 inhibitors in comparison to classic NSAIDs, which is its smaller tendency to produce adverse gastrointestinal effects.

More recently there has been a meta-analysis with a higher number of patients analyzed, that concluded that celecoxib is safer (less incidence of ulcers and complications) than classic NSAIDs. These results obtained through the different meta-analyses have been corroborated in daily clinical practice and several observational studies, either of cohorts or of cases and controls. In both, COXIBs have been associated with a lower risk of hemorrhage than with conventional NSAIDs.

This assertion is endorsed by the CLASS (Celecoxib Long-term Arthritis Safety Study) that included about 8,000 patients with arthrosis or rheumatoid arthritis (RA) where 20% of the participants took ASA at a low dose for cardiovascular prophylaxis. The GI toxicity was assessed after 6 months of treatment with celecoxib (400 mg every 12 hours) in comparison to ibuprofen (800 mg every 8 hours) Diclofenac (75 mg every 12 hours). The incidence of ulcer complications (perforation, obstruction and bleeding), the main variable of the study, was not significantly different. However, celecoxib was associated with a lower incidence of GI hemorrhage in patients that did not take ASA at the same time. An effective half-life of between 8 and 12 hours has been estimated for celecoxib, in accordance with the datasheet in the FDA.

On the other hand, we have diacerein with its chemical name 4,5-Bis(acetyloxy)-9,10-dihydro-9,10-dioxo-2-anthracenecarboxylic acid, represented by formula (II),

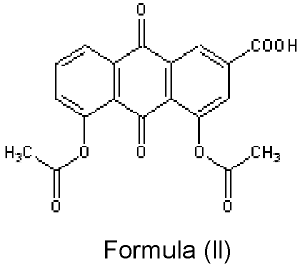

Described for the first time in US 4,244,968 for the treatment of arthritis.

It is an anthraquinone derivative with its active metabolite being rhein and having similarities in its chemical structure to tetracyclines. It has been used in disorders of the musculoskeletal system and of joints as an interleukin-1β inhibitor, producing anti-inflammatory effects and anabolic effects by promoting the production of TGF-β.

The anti-arthrosic properties of diacerein are thanks to its ability to inhibit pro-inflammatory and pro-catabolic cytokines such as interleukin-1β which performs an important role in the degradation of articular cartilage, as well as in the inhibition of the production and release of enzymes that degrade cartilage, (collagenase and stromelysin) without affecting the synthesis of prostaglandins.

Diacerein, through its active metabolite, rhein, is specifically indicated in those diseases that involve abnormalities of the articular cartilage in patients with osteoarthritis, rheumatoid arthritis, ankylosing spondylitis and other less common diseases.

In animal models, diacerein has shown beneficial effects on the cartilage to prevent or lessen macroscopic and microscopic injuries in articular tissue. In clinical studies of from 2 to 6 months long, diacerein was found to significantly reduce, in comparison to the placebo, pain and functional impairment in patients with OA of the hip and knee.

Even with a carryover effect as after a treatment of 3 months with diacerein, a statistically significant improvement in terms of the pain and function was observed when compared with the placebo group, for up to two months after the last administration, which situates diacerein as a symptomatic slow-acting drug for osteoarthritis (SYSADOA) that is not only effective in improving the symptoms but also reduces the degradation of cartilage.

Reports on meta-analyses of controlled clinical trials with diacerein show its superiority to the placebo for improving pain relief and function, with a similar effect to NSAIDs, although it is better than both in the follow-up period, by maintaining its beneficial effect for up to 3 months with good tolerability.

An increase has been demonstrated in the bone remodeling process, with the possibility that diacerein also has an influence by reducing the resorption of subchondral bone.

This invention is characterized by providing a composition that comprises the combination of a selective NSAID inhibitor of cyclooxygenase-2 and an anthraquinone derivative, more specifically the combination of celecoxib with diacerein. A combination capable of providing long-term treatment for the pain and inflammation of osteoarthritis, rheumatoid arthritis and/or degenerative joint disease.

In the state of the art, the patent US 7,387,623 describes the manufacturing of an article and its use in administering a pharmaceutical composition in the form of a parenteral suspension by intramuscular, subcutaneous or intradermal injection, that comprises a vial that has (a) a first chamber filled with the aqueous suspension that comprises (i) an aqueous medium; (ii) a drug in the form of solid particles in a therapeutically effective selected amount of celecoxib and diacerein among other steroid drugs; and (iii) one or more humectant and/or suspensor agents in an effective amount to provide a controlled flocculation of the drug, with at least one ingredient of the formulation being liable to oxidative degradation; (b) a second chamber that is substantially empty but for a gaseous medium; (c) a septum that separates the first and second chamber and makes them impermeable to the gaseous medium; the patent US 9,408,806 describes forms of dosage of two-layered tablets and methods for preparing thereof, that can be used for different classes of active pharmaceutical ingredients selected from celecoxib and diacerein among others with pharmaceutically acceptable inert excipients such as agglutinating agents, fillers, anti-oxidants, disintegrants, surfactants, lubricants and glidants, present in a fast-release, delayed-release, sustained-release, prolonged-release, controlled-release or modified-release form; the patent US 9,737,490 describes a form of pharmaceutical dosage that comprises (i) at least one formed segment (S1), that contains a first pharmacologically active ingredient (A1) such as opioids and provides a controlled release thereof, and (ii) to at least one additional segment (S2), that contains a second pharmacologically active ingredient (A2) selected from celecoxib, diacerein, among other drugs and provides a fast-release thereof, for treating pain in a patient; the patent US 9,814,712 describes a method for treating pain that comprises: administer an (S) enantiomer of mannitol or one of its pharmaceutically acceptable salts such as pure (S)-pirilindol enantiomer and at least one additional analgesic agent that is selected from celecoxib and diacerein.

This invention is characterized by providing a composition that comprises the combination of celecoxib or its pharmaceutically acceptable salts with diacerein or its pharmaceutically acceptable salts, not reported in the state of the art. The potential advantage of using the therapy of said combination is that the analgesic and antiarthritic effects can be maximized, while the incidence of adverse effects is minimized. In the case of the combination of celecoxib with diacerein, diacerein on its own does not generate initial painkilling effects while the combination with celecoxib presents greater analgesic effectiveness than that shown by celecoxib when administered in individual doses. The combination of celecoxib with diacerein makes it possible to reduce the pain and inflammation as well as to restore function, permitting the patient a prompt return to their daily activities. The use of this combination of drugs offers an analgesic synergy that permits a reduction in the doses required together with a reduction in the adverse effects.

### OBJECT OF THE INVENTION

To offer a new therapeutic option for the long-term control and treatment of pain and inflammation in osteoarthritis, rheumatoid arthritis and/or degenerative joint disease that manages to lessen symptoms and improve the quality of life of patients. The utility of the combination of celecoxib with diacerein, which are administered with pharmaceutically acceptable excipients or adjuvants, formulated in a single dosing unit to be administered orally, generates synergic interaction, increasing its therapeutic potency, onset of action and reduction of adverse events.

The combination of said active ingredients results in a higher pharmacological potency, improving the therapy, offering benefits such as: administration of weaker concentrations of the active ingredients that when they are administered separately, higher effectiveness and greater therapeutic potency, apart from significantly lowering the probability of side effects that can arise when they are administered independently in comparison to when they are administered separately. Said combination has enhanced properties and an additional antiarthritic effect.

### BRIEF DESCRIPTION OF THE FIGURES

Figure 1. Temporary courses (TCs) of antinociceptive effects generated by oral celecoxib in the experimental model of "Pain-induced functional impairment model in the rat" where a "gout" type pain has been established. There is a dose-dependent ratio.
Figure 2. Dose/response curve (CDR) for oral celecoxib by plotting the overall antinociceptive effects as the area under the curve (AUC) for the corresponding temporary courses against the administered doses of the drug. The standard mean ± error is plotted.
Figure 3. Temporary courses of antinociceptive effects generated by the combination of celecoxib with diacerein of 50 mg/kg orally in the experimental model of "Pain-induced functional impairment model in the rat", in acute administration.
Figure 4. Temporary courses (CT) of antinociceptive effects generated by the combination of celecoxib with diacerein of 50 mg/kg orally in rats that received, in chronic form, the combination of celecoxib 100 mg/kg with diacerein 50 mg/kg, in the experimental model of "Pain-induced functional impairment model in the rat".
Figure 5. Temporary courses of antinociceptive effects generated by the most effective doses of the combination of celecoxib with diacerein 50 mg/kg, in acute and chronic treatment in the experimental model of "Pain-induced functional impairment model in the rat".
Figure 6. Overall antinociceptive effects such as AUC generated by the combination of celecoxib 1000 mg/kg with diacerein 50 mg/kg by itself (acute T.), and the effects generated by the combination of celecoxib 100 mg/kg with diacerein 50 mg/kg by itself (chronic T.) orally in the experimental model of "Pain-induced functional impairment model in the rat".
Figure 7. CDR (death) at 24 h post-treatment with diacerein by itself, celecoxib by itself and the combination of celecoxib with diacerein 50 mg/kg.
Figure 8. CDR (death) at 48 h post-treatment with diacerein by itself, celecoxib by itself and the combination of celecoxib with diacerein 50 mg/kg.
Figure 9. CDR (death) at 72 h post-treatment with diacerein by itself, celecoxib by itself and the combination of celecoxib with diacerein 50 mg/kg.

### DETAILED DESCRIPTION OF THE INVENTION

The management of pain and inflammation in osteoarthritis, rheumatoid arthritis and/or degenerative joint disease is known to be complex and the response to existing treatments is insufficient, even with drugs that have been established on the basis of consensual recommendations, effectiveness is unpredictable, the dosage can be complicated and adverse effects are common. This invention has demonstrated with preclinical tests that the novel combination of celecoxib with diacerein in specific dosages shows an unexpected and strong therapeutic synergic effect in the treatment of pain and inflammation in osteoarthritis, rheumatoid arthritis and/or degenerative joint disease; so the main aim of this invention is the development of a pharmaceutical composition comprising the combination of a selective inhibitor of cyclooxygenase-2, made up by the active ingredient celecoxib or its pharmaceutically acceptable salts and an anthraquinone derivative made up by the active ingredient diacerein or one of its pharmaceutically acceptable salts, which are administered with pharmaceutically acceptable excipients or adjuvants, formulated in a single dosing unit to be administered orally, which is indicated for the control and treatment of pain and inflammation in osteoarthritis, rheumatoid arthritis and/or degenerative joint disease. Moreover, the combination presents an additional anti-arthrosic effect.

One currently available alternative for increasing the effectiveness of an analgesic treatment and significantly lowering the side effects is through the administration in combination of two or more active agents, such as the synergistic drug combination whose protection is being sought in this invention.

This invention seeks to provide a new therapeutic option for the control and treatment of pain and inflammation in osteoarthritis, rheumatoid arthritis and/or degenerative joint disease, that manages to reduce the patients' symptoms and improve their quality of life. The combination of said active ingredients results in a higher pharmacological potency.

At the present time the effects that can be produced by the combination of celecoxib with diacerein in pain and inflammation in osteoarthritis, rheumatoid arthritis and/or degenerative joint disease has not been determined. For which, the determination and assessment were performed in this work for the antinociceptive effect of the individual and combined administration of celecoxib and diacerein, in order to determine their individual effects and the type of synergic interaction shown by these drugs in a preclinical model where it is possible to establish arthritis of the gout type in rats and compare the antinociceptive effectiveness of a variety of individual drugs or drugs in combination in the animals with this alteration, as well as determining possible adverse effects that said association of drugs could cause.

Said preclinical model refers to the PIFIR experimental model (Lopez-Muñoz and col., 1993) where it is possible to establish gout in rats and compare the antinociceptive effectiveness of a variety of individual drugs or drugs in combination in the animals with this alteration. This experimental model has some significant advantages over other preclinical methodologies: a) establishes an alteration in rats that clinically affects humans: gout; b) the temporary course of the analgesic or antinociceptive effect can be determined for up to 4 continuous hours in the same experimental subject, without producing conditioning or learning in the rat, which is a significant advantage in comparison with other experimental models; c) less test compound is consumed than in other experimental models that use larger animal species; d) the assessment of the antinociceptive effects is done by means of a computer and does not depend on the sometimes subjective observations of the assessor. As already mentioned, the PIFIR model has been very useful for studying the antinociceptive effects of analgesic compounds by themselves as well as of combinations of analgesic compounds, for studying the mechanisms of action of analgesic compounds, such as the combination of celecoxib with diacerein, for which invention protection is sought.

The therapy is improved with said combination, offering benefits such as: administration of weaker concentrations of the active ingredients that, when they are administered separately, higher effectiveness and greater therapeutic potency, apart from significantly lowering the probability of side effects that can arise when they are administered independently in comparison to when they are administer separately. Moreover, there is a reduction in the side-effects that the separate administration of each compound could cause, through lower doses from the ones employed commercially. Therefore, the behavior of celecoxib in combination with diacerein was preclinically demonstrated, managing to determine the interaction and synergy between both of them together with the optimal combination proportions and a high degree of therapeutic effectiveness and enhancement.

As a result of the above, the assessment was carried out of the antinociceptive effect of individual administration in the case of celecoxib and in the case of combined administration of celecoxib with diacerein, owing to the fact that diacerein by itself does not show any evidence of antinociceptive effects.

### Experimental model

### Animals for experimentation

In order to determine possible antinociceptive effects and toxic effects, male Wistar rats [Crl:(WI)BR] were employed with a weight of between 180 and 200 g. All the experimental procedures followed the recommendations of the Committee for Research and Ethical Issues of the International Association for the Study of Pain and the Guidelines on Ethical Standards for Investigations of Experiment Pain in Animals. The number of animals for experimentation was kept to a minimum (6 animals per experimental point for antinociceptive effects and 10 animals per experimental point for adverse effects: lethality). The animals were kept in a room with alternating dark/light cycles. Twelve hours before the antinociception experiments, food was withdrawn from the rats, only leaving them free access to water. All the experiments were carried out during the light phase.

### Compounds for experimentation

The following compounds were employed: uric acid, celecoxib and diacerein, propylene glycol, carboxymethyl cellulose and mineral oil. The celecoxib and diacerein were dissolved in propylene glycol (5%) and carboxymethyl cellulose.

### Assessment of antinociceptive activity

It started with determining the antinociceptive effects of the individual compounds to then go on to analyze the effects of the combination, employing the "Pain-induced functional impairment model in the rat" ("PIFIR model"). The animals were anesthetized in a glass desiccator, saturated with ether vapor. The gout was induced by applying an intra-articular injection (i.a.) of 0.05 ml of uric acid suspended in mineral oil at 30% in the right hind member, precisely in the femur-tibia-patella joint.

A 1 mL glass syringe with a 4-mm-long No. 22 needle was used for the intra-articular injection. Immediately afterwards, an electrode is attached to each hind paw (of the rats) in the middle of the plantar calluses. The rats were left to recover from the anesthesia and placed in a 30-cm-diameter rotary stainless-steel cylinder. The cylinder was turned at 4 r.p.m., forcing the rats to walk for 2 min every half hour, for a total of 6.5 hours, (2.5 h to generate the arthritis and 4 h for the assessment of antinociceptive effects). The variable measured was the contact time of each one of the rats' hind legs in the cylinder.

When the electrode makes contact with the cylinder a circuit is closed and the ratio between the contact time of the leg administered with uric acid in respect of the one that did not have uric acid administered was recorded on a computer.

### Assessment of toxic effects (lethality).

For the preclinical toxicity studies (lethality), the range of oral doses was first determined for the individual compounds (celecoxib by itself, and diacerein by itself), from the maximum dose that does not produce death, to the minimum dose required to 100% precipitate death in the population of laboratory animals (rats) that receive the treatment. The toxic effects of death in single administrations were determined at 3 different post-treatment times: 24h, 48h and 72h, to be able to determine the corresponding DRC (death). In the determinations presented for the individual drugs or drugs in combination, an attempt was made to administer the treatment on a single occasion but, when this was not possible owing to the high concentration and limits of solubility, several administrations were made with 3-hour time intervals. The established doses were always increased in logarithmic units administered orally both for celecoxib and for diacerein.

### Experimental protocol.

First it was decided to determine the range of doses that could form the celecoxib dose/response curve. It has been already demonstrated in this same experimental model and the same experimental conditions for diacerein that they do not provide evidence of antinociceptive effects. The 50 mg/kg dose of diacerein has been determined to be a proper dose in the preclinical stage to be employed in combination and be able to determine if changes are produced in the antinociceptive effects.

Thus, the possible antinociceptive effects of both the individual and the combined forms of the compounds were determined and analyzed using animals with gout in the experimental model of Pain-induced functional impairment model in the rat. The determination was carried out under 2 conditions: 1) Acute administration in animals with gout, 2) Chronic administration of the drugs (a daily administration for 7 days) and determination of the antinociceptive effects in animals in which gout was generated.

After the uric acid was administered to the joint to produce the gout type impairment and alteration, there was a wait of 2.5 hours for the total impairment to be produced (the symptoms of gout were already complete at 2.5 hours after the uric acid was administered). This time (2.5 hours after the administration of the uric acid) was considered to be "0" time, for administering the treatment, at this moment, with the individual drugs and the drugs in combined form, followed by the temporary courses of each treatment being determined for the following 4 hours. An "n" of 6 rats were employed per treatment. For the purpose of this study, inducing harm in the experimental animals was unavoidable. However, care was taken to avoid causing unnecessary suffering to the animals.

Having said that, the possible effects that celecoxib by itself or the combination of celecoxib with diacerein 50 mg/kg could generate were determined, in treatments in acute forms, and a similar procedure was employed, but in animals that received either 100 mg/kg celecoxib or the combination of 100 mg/kg celecoxib with 50 mg/kg diacerein during 7 days. Each one of the compounds was administered orally to the rats in a volume of 20 ml/kg.

From the afore-described that corresponds to the experimental model of preclinical analysis of this combination of celecoxib with diacerein whose protection is being sought, the following results were obtained: The data are expressed as a percentage Functionality Index (FI%). This FI% is the ratio obtained by dividing the contact time of the limb with uric acid by the contact time of the limb against of the same rats, and multiplying the result by 100. The temporary course (TC) curves are built by plotting FI% against time (h); under these experimental conditions, the analgesic or antinociceptive effect is estimated as the recuperation of FI%. All the values plotted in the figures correspond to the mean ± standard error for 6 animals.

The uric acid at 20% induced a full impairment of the right hind leg approximately 2.5 h after administration, this corresponded to a value of zero for the FI%. At that time, the rats that only received the vehicle (5% propylene glycol in carboxymethyl cellulose) administered orally (negative control) did not show any recovery of the FI% during the following 4-hour assessment period. In all the graphs, the mean and standard error is presented for the assessment of antinociceptive effects after "0" time, which is the moment when the arthritis is completely developed in the rats and is the precise time for the administration of the drugs.

The animals with gout that were administered with diacerein in 50 mg/kg oral doses did not show any antinociceptive effect whatsoever during the 4 h of assessment.

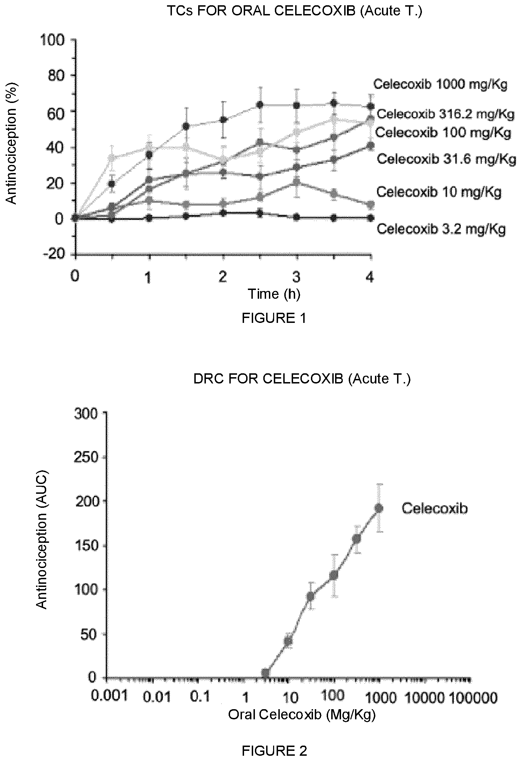

Figure 1 presents the Temporary Courses (TC) developed for celecoxib in single or acute administration for 4 hours in doses with logarithmic increments (from 3.2 to 1000 mg/kg). X axis is the assessment time in hours, while the Y axis is the antinociceptive effect assessed as a functionality index percentage (FI%). In "0" time (the moment when the rats already have total impairment or cannot use the limb when walking) the celecoxib was administered in an individual administration in different doses. Under these experimental conditions, dose-dependent antinociceptive effects during the 4 hours of observation were proved.

Then, to determine the overall antinociceptive effects generated by every different dose of celecoxib, the area under the curve (AUC) was calculated for each individual temporary course (TC), which not only shows the effect over the 4 h of assessment but also includes the maximum effects generated over time. With these AUC, the dose-response curve (DRC) was built for celecoxib, which is shown in figure 2. The "X" axis in this figure indicates the doses in logarithmic increments, while the "Y" axis shows the overall antinociceptive effects (AUC) plotted to get the DRC. The mean and standard error was plotted and the complete sigmoid corresponding to the DRC for orally administered celecoxib was observed. The maximum antinociceptive effectiveness reached was 191.9 ± 27.2 area units (au).

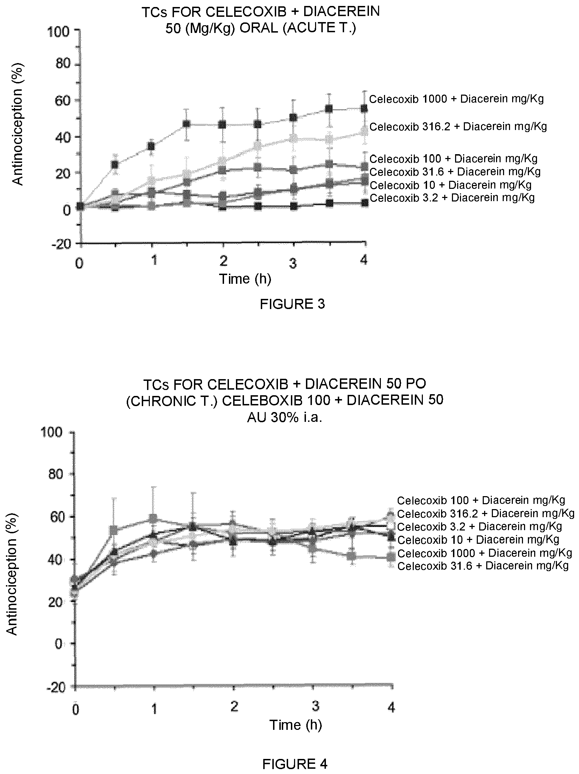

Afterwards the temporary courses of celecoxib were determined but simultaneously administering diacerein in 50 mg/kg oral doses in acute treatment. The axes correspond to the ones described for figure 1 and it can be observed that dose-dependent effects were also obtained (figure 3).

In the case of the DRC for the combination of celecoxib with diacerein 50 in rats with chronic treatment of 100 mg/kg celecoxib with 50 mg/kg diacerein, the temporary courses (TC) of celecoxib with 50 mg/kg diacerein were first plotted and are shown in figure 4. The mean of n=6 and standard error at each point are shown plotted in the figure.

It is observed that celecoxib in different doses as well as associated with diacerein developed an antinociceptive effect in the rats that chronically received 100 mg/kg celecoxib with 50 mg/kg diacerein. As the doses increase, the antinociceptive effects increase slightly. Once again, it is worth mentioning that, given that the rats are receiving the analgesic treatment in chronic and repeated form, even when they were administered the 8° day with the intra-articular uric acid, the impairment of the limb did not reach 0%. But anyway, it is possible to detect and assess the antinociceptive effects of the chronic treatment during 4 hours.

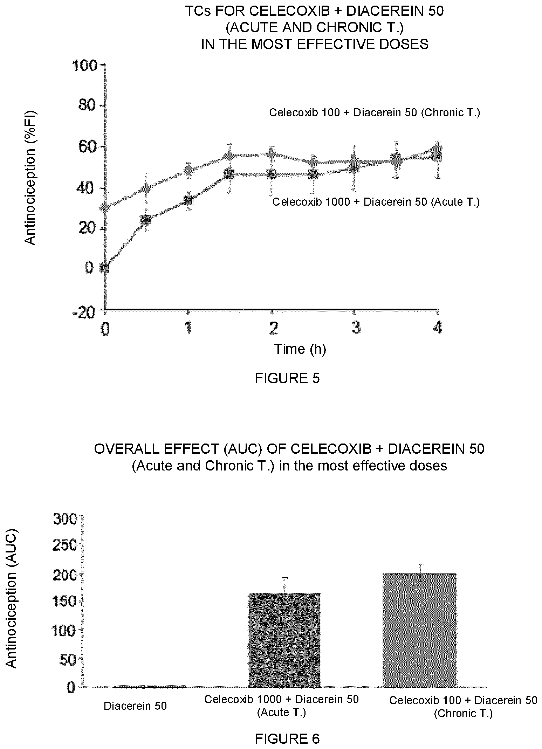

Figure 5 presents the TC for the most effective doses of both treatments with celecoxib+diacerein 50 in acute and chronic treatment. There is evidence that antinociceptive effects can be produced with smaller doses (100 mg/kg celecoxib with 50 mg/kg diacerein) but in chronic treatment, that tend to be more effective than the antinociceptive effects generated by a higher dose of celecoxib (1000 mg/kg celecoxib + 50 mg/kg diacerein) in acute administration.

Figure 6 presents the overall antinociceptive effects of the TC shown in figure 5, but now as AUC: 1000 mg/kg celecoxib with 50 mg/kg diacerein for acute treatment produces an effect of 163.3 ± 28.6 au, while 100 mg/kg celecoxib with 50 mg/kg diacerein after chronic treatment produces an effect of 200.6 ± 14.7 au. In other words, better antinociceptive effectiveness can be achieved with a smaller amount of drug (100 mg/kg celecoxib with 50 mg/kg diacerein) but in chronic treatment.

As regards the analysis of toxicity (death) generated by the combination of celecoxib with diacerein, for the preclinical toxicity studies, as has already been mentioned, the range of oral doses was first determined from the maximum dose that does not produce death, to the minimum dose necessary to produce 100% death in the population that receives treatment for celecoxib by itself, diacerein by itself and for the combination of celecoxib with diacerein 50 mg/kg. The toxic effects of death were determined at 3 different post-treatment times: 24, 48 and 72 hours, to be able to determine the corresponding DRC (death). In the determinations presented below, the treatment was administered on a single occasion and, in the case of celecoxib, high doses in several administrations until the required dose is completed. The established doses were always increased in logarithmic units and administered orally.

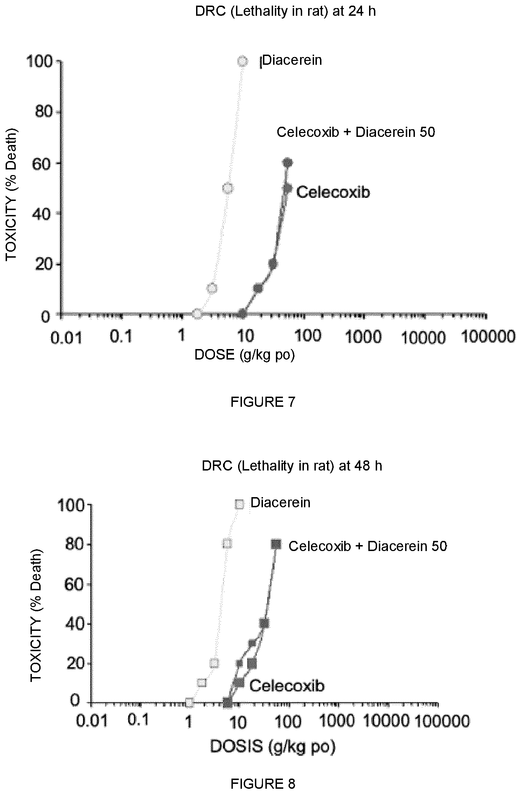

Figure 7 shows the joint DRC for diacerein, celecoxib and the combination of celecoxib with 50 mg/kg diacerein in rats 24 hours after the administration. The complete DRC can be observed for the lethality of diacerein and only one part of the DRC for the lethality of celecoxib by itself and the combination of celecoxib with 50 mg/kg diacerein, as a limit was reached for the maximum concentration to be administered. It can also be pointed out that the doses required to start to produce effects of lethality are a long way away from the doses that form the combination: in the case of diacerein the proposed dose of 50 mg/kg is a long way away from the dose that starts to generate effects of lethality at 24 hours: 3,162 mg/kg (3.16 g/kg) While in the case of celecoxib, the maximum dose in the DRC for antinociceptive effects is 1 g/kg, while the DRC for lethality starts with 17.8 g/kg, which is also a long way away from the dose that generates the desired effects.

Figure 8, shows the joint DRC for diacerein, celecoxib and the combination of celecoxib with 50 mg/kg diacerein in rats 48 hours after administration. The complete DRC can be observed for the lethality of diacerein and only part of the DRC for the lethality of celecoxib by itself and the combination of celecoxib with 50 mg/kg diacerein, as a limit was reached for the maximum concentration to be administered. It can also be pointed out that the doses required to start to produce effects of lethality are a long way away from the doses that form the combination: in the case of diacerein, the proposed 50 mg/kg dose is a long way away from the dose that starts to generate effects of lethality at 48 hours: 1.8 g/kg. While in the case of celecoxib, the maximum dose in the DRC for antinociceptive effects is 1 g/kg, while the DRC for lethality at 48 hours starts with 10 g/kg, which is also a long way away from the dose that generates the desired effects.

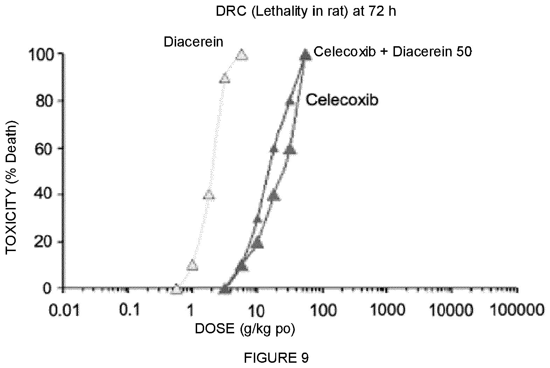

Figure 9, shows the joint DRC for diacerein, for celecoxib and the combination of celecoxib with 50 mg/kg diacerein in rats 72 hours after administration. The complete DRCs can be observed for the lethality of diacerein, celecoxib by itself and the combination of celecoxib with 50 mg/kg diacerein. It can also be pointed out that the doses required to start to produce effects of lethality are a long way away from the doses that form the combination: in the case of diacerein, the proposed 50 mg/kg dose is a long way away from the dose that starts to generate effects of lethality at 72 hours: 1.0 g/kg. While in the case of celecoxib the maximum dose in the DRC for antinociceptive effects is 1.0 g/kg, while the DRC for lethality at 72 h starts with 5.6 g/kg, which is also a long way away from the dose that generates the desired effects.

In the current state of the art, there are pharmacological treatments for pain, however, there is no one treatment that is characterized by the combination of the active agents, celecoxib with diacerein, which is why the development of this invention provides a current safe alternative for the control and treatment of neuropathic pain, managing to lower treatment times, therapeutic effects and secondary reactions. The administration of said compounds given by each one is an amount of approximately 0.01 mg to approximately 1000 mg a day of treatment of celecoxib is given for each one, while diacerein is given in an amount of approximately 0.01 mg to approximately 500 mg a day.

This invention is developed for oral, nasal, intramuscular, intravenous, and topical administration; either in the form of fast release for both drugs or modified release for one or both drugs, with a smaller dose, there is greater therapeutic potency and a lower risk of adverse events.

### EXAMPLES

A description is given below, by way of illustration and not as a limitation, of some pharmaceutical compositions:

### Example 1: Compositions for oral, nasal and/or topical administration

| |
|---|
| Celecoxib or pharmaceutically acceptable salt |
| Diacerein or pharmaceutically acceptable salt |
| Pharmaceutically acceptable excipient and/or vehicle and/or additive |

### Example 2: Composition for intramuscular and intravenous administration

| |
|---|
| Celecoxib or pharmaceutically acceptable salt |
| Diacerein or pharmaceutically acceptable salt |
| Pharmaceutically acceptable excipient and/or vehicle and/or additive |

This invention can be represented in other specific forms while staying in keeping with its spirit or respecting its essential characteristics and the excipients and/or vehicles and/or additives to obtain the desired pharmaceutical form are selected from: polyvidone K30, colloidal silicon dioxide, croscarmellose sodium, microcrystalline cellulose PH 102, sodium starch glycolate, magnesium stearate, sucrose, talc, vaseline, paraffin, polyethylene glycol, wax, silicon, anhydrous absorption bases, hydrophilic additives, anhydrous emulsifying bases, zinc oxide, war rubber, sorbitol, lactose, microcrystalline cellulose, calcium carbonate, cellulose acetate, glycerin, hypromellose, lanolin, maltodextrina, pectin, polydextrose, pregelatinized starch, sodium lauryl sulfate, sucralose, eudragit, HPMC, HMC, purified water, 90° ethyl alcohol, methyl alcohol. The methods described shall, in all their aspects, only be treated as examples and not as restrictions. Therefore, the scope of this invention is given in the attached claims rather than in the above description. Its scope shall include all the changes that fall within the meaning and range of equivalence of the claims.

Overall, this invention has the following advantages:
1. The chronic treatment with "celecoxib" generated better antinociceptive effects than those generated by celecoxib when it is administered in acute form.
2. The chronic treatment with the "celecoxib with diacerein" combination generated more antinociceptive effects than the combination of celecoxib with diacerein when it is administered in acute form.
3. The DRC for the effects of lethality of diacerein is more to the left of that of celecoxib, showing that diacerein requires smaller ranges of doses to produce the lethality effect.
4. The DRC for the lethal effects of the combination of celecoxib with diacerein shifts slightly to the left in relation to the DRC for the lethal effects shown by celecoxib by itself. This translates into the fact that the combination could generate effects of lethality with smaller doses than when celecoxib is used by itself.
5. To generate effects of lethality with diacerein by itself, celecoxib by itself or the combination of celecoxib with diacerein, it is necessary to administer extremely high doses, far removed from the therapeutic doses.

## Claims

1. A synergic pharmaceutical combination **characterized in that** it comprises:
i. a selective COX-2 inhibitor agent and/or pharmaceutically acceptable salts thereof,
ii. an anthraquinone derivative agent and/or pharmaceutically acceptable salts thereof,
iii. a pharmaceutically acceptable vehicle and/or excipient,
which are formulated in a single dosing unit for oral, parenteral, transdermal and topical administration, which is indicated for the long-term control and treatment of pain and inflammation in osteoarthritis, rheumatoid arthritis and/or degenerative joint disease and an additional anti-arthrosic effect in mammals; wherein the selective COX-2 inhibitor is preferably celecoxib or pharmaceutically acceptable salts thereof and the anthraquinone derivative agent is preferably diacerein or pharmaceutically acceptable salts thereof.

2. The combination of claim 1, **characterized in that** the active agent celecoxib is in a concentration of from approximately 0.01 to approximately 1000 mg.

3. The combination of claim 1, **characterized in that** the active agent diacerein, is in a concentration of from approximately 0.01 to approximately 500 mg.

4. The combination of claim 1, useful for the preparation of a drug product for pain and inflammation therapy in osteoarthritis, rheumatoid arthritis and/or degenerative joint disease with an additional anti-arthrosic effect in mammals.

5. The combination of claim 1, useful for the preparation of a drug product that is formulated in a single dosing unit to be administered orally in the form of capsules, tablets, pills, sublingual tablets, granules, caplets, suspensions or solutions. In both intramuscular and intravenous injectable form and in the form of topical patches, ointments, gels and creams. As well as in suppositories or transdermal, oral or nasal inhalation devices.

6. The combination of claim 1, wherein mammal refers to a human being or an animal.

7. A treatment method for the pain and inflammation of osteoarthritis, rheumatoid arthritis and/or degenerative joint disease that comprises administering to mammals that suffer from said disease an effective amount of the combination of at least one compound selected from the group of selective COX-2 inhibitors, preferably celecoxib or pharmaceutically acceptable salts thereof and at least one compound selected from the group of anthraquinone derivatives, preferably diacerein or pharmaceutically acceptable salts thereof.

8. A treatment method for neuropathic and/or nociceptive pain according to claim 7, wherein the compounds are administered by oral, nasal, topical, intramuscular and/or intravenous means.

9. A method for the treatment of pain and inflammation in osteoarthritis, rheumatoid arthritis and/or degenerative joint disease according to claim 8, wherein the compounds are administered in amounts of from approximately 0.01 mg to approximately 1000 mg a day of celecoxib or the pharmaceutically acceptable salts thereof and from approximately 0.01 mg to approximately 500 mg a day of diacerein or the pharmaceutically acceptable salts thereof.
